(19) Europäisches Patentamt / European Patent Office / Office européen des brevets

(11) **EP 3 425 636 A1**

(12) **EUROPÄISCHE PATENTANMELDUNG**

(43) Veröffentlichungstag:
**09.01.2019 Patentblatt 2019/02**

(51) Int Cl.:
*G16H 50/20* *(2018.01)*

(21) Anmeldenummer: **17179899.4**

(22) Anmeldetag: **06.07.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Justus-Liebig-Universität Gießen 35390 Gießen (DE)**

(72) Erfinder: **Dr. Wilbrand, Jan-Falco 35392 Gießen (DE)**

(74) Vertreter: **Stumpf, Peter c/o TransMIT GmbH Kerkrader Strasse 3 35394 Gießen (DE)**

(54) **VERFAHREN UND VORRICHTUNG ZUR EINSTUFUNG DER KOPFFORM**

(57)     Die Erfindung betrifft ein Verfahren zur Einstufung der Schädelform. Dabei wird zunächst die Länge wenigstens eines Paares der Schädeldiagonalen $SD\alpha1$ und $SD\alpha2$ erfasst. Dann wird wenigstens einer der Kopfparameter Diag_Ratio_$\alpha$ und/oder CVA und/oder CVAI aus der Länge wenigstens eines Paares der Schädeldiagonalen $SD\alpha1$ und $SD\alpha2$ berechnet. Dann erfolgt die Einstufung der Schädelform je nach Abweichung des/der Kopfparameter von Kopfparameternormwerten gemäß normativen Perzentilen in eine von wenigstens 3 Kategorien, wobei die Schädelform in die Kategorie "normal" eingestuft wird, wenn alle Werte der Kopfparameter zwischen der 10% und der 90% Perzentile liegen, die Schädelform in die Kategorie "moderat deformiert" eingestuft wird, wenn wenigstens ein Wert außerhalb der 10% und der 90% Perzentile liegt, aber alle Werte der Kopfparameter zwischen der 3% und der 97% Perzentile liegen oder in die Kategorie "schwer deformiert" eingestuft wird, wenn wenigstens ein Kopfparameter außerhalb der 3% und der 97% Perzentile liegt.

**Fig.1**

EP 3 425 636 A1

**Beschreibung**

Die vorliegende Erfindung betrifft ein Verfahren und eine Vorrichtung zur Einstufung der Form eines Kopfes. Einstufung heißt hierbei, dass die Kopfform hinsichtlich des Vorliegens einer Schädeldeformität beurteilt wird.

**Beschreibung und Einleitung des allgemeinen Gebietes der Erfindung**

Seit der Empfehlung der kinderärztlichen Gesellschaften zur ausschließlichen Rückenlagerung von Säuglingen innerhalb des ersten Lebensjahres nehmen kindliche Schädeldeformitäten ("Liegeschädeldeformitäten") massiv an Zahl zu. Aktuell gehen Fachleute von einer Häufigkeit von 1:60 aus. Dies bedeutet, dass eine erste Erfassung der kindlichen Kopfform aktuell sehr häufig notwendig wird, um behandlungsbedürftige Schädeldeformitäten zu erkennen und einer Therapie zuzuführen. Ca. 30% aller Säuglinge zeigen in den ersten 2 Monaten eine passagere Rumpfasymmetrie, die sich auch auf die Schädel- und damit die Kopfform auswirken kann Die Umsetzung der Empfehlung, Säuglinge auf dem Rücken zu lagern, führte zu einem deutlichen Rückgang der plötzlichen Todesfälle (SIDS). Diese "back to sleep" Kampagne hat nachweislich zu einem höheren Prozentsatz an Lagerungsasymmetrien des kindlichen Kopfes geführt.

**Stand der Technik**

Daten zur anthropometrischen Schädelvermessung werden an spezialisierten Zentren erhoben. Dabei unterscheiden sich die Messmethoden der einzelnen Zentren deutlich voneinander. So kommen zum Beispiel manuelle anthropometrische Messungen der Schädellänge, -breite und der Diagonalen mit Maßband und Messzirkel zur Anwendung. Dies erfordert in diesen Messungen geschultes Personal. Alternativ sind Schädelmessungen mit 3D-Photogrammetrie möglich, was einen sehr hohen apparativen und monetären Aufwand erfordert. Ebenfalls sind Schädelmessungen mit Laser, mittels thermoplastischen oder silikonbasierten Kurvenlinealen möglich.

Die relevantesten Kopfparameter sind Schädellänge, Schädelbreite und die tanskraniellen Diagonalen. Die Schädelasymmetrie wird aus der Länge eines Paares der Schädeldiagonalen $SD\alpha 1$ und $SD\alpha 2$ bestimmt.

Ein Paar der Schädeldiagonalen $SD\alpha 1$ und $SD\alpha 2$ sind dabei zwei Diagonalen, die auf den knöchernen Schädel projiziert durch den Kreuzungspunkt von Längs- und Querdurchmesser des Schädels gehen und jeweils einen festen Winkel $\alpha$ haben, der üblicherweise um etwa 30 Grad vom Längsdurchmesser des Schädels abweicht.

Das Diagonalenverhältnis Diag_Ratio_$\alpha$ ist das Verhältnis eines Paares der Schädeldiagonalen ($S\alpha$_Diag A / $S\alpha$_Diag B), wobei ein Ergebnis von 1 eine vollkommene Symmetrie darstellen würde.

Die Cranial Vault Asymmetry (CVA) beschreibt den absoluten Längenunterschied eines Paares der Schädeldiagonalen ($S\alpha$_Diag A -Sa_Diag B).

Der Cranial Vault Asymmetry Index (CVAI) beschreibt das Verhältnis des absoluten Längenunterschiedes zwischen einem Paar der Schädeldiagonalen geteilt durch die Länge der kürzeren Schädeldiagonalen multipliziert mit 100.

$$CVAI\alpha = \frac{|S\alpha\_Diag\_A - S\alpha\_Diag\_B|}{S\alpha\_Diag\_A} x100$$

Es erfolgt aktuell die Vorstellung von Kindern, bei denen eine behandlungsbedürftige Schädeldeformität vermutet wird, zunächst bei diversen medizinischen und paramedizinischen Fachleuten, bis schließlich die Überweisung an ein spezialisiertes Zentrum erfolgt. Hier wird die Schädelform i.d.R. standardisiert erfasst und ggf. therapeutische Maßnahmen eingeleitet. Für Betroffene sind damit bisher ein langer Anfahrtsweg und z.T. lange Wartezeiten auf Termine verbunden. Weiterhin kann die Entwicklung / der klinische Verlauf einer Schädeldeformität während einer Therapie nur bei neuerlichen Vorstellungsterminen im spezialisierten Zentrum erneut erfasst werden.

All den bislang verwendeten Messmethoden ist gemeinsam, dass eine universelle (d.h. für jede Person) und ortsunabhängige Erfassung der Schädelform nicht möglich ist. Die wissenschaftliche Auswertung der Daten aus unterschiedlichen Behandlungszentren folgt uneinheitlichen Messprotokollen und lässt so nur eine begrenzte Vergleichbarkeit zu. Dies bedingt Mängel in der Studienlage zum Thema. Eine erste Einschätzung der Schädelform, aber auch die Kontrolle des klinischen Verlaufs bleibt Standort- und Fachpersonalgebunden.

**Aufgabe**

Aufgabe der vorliegenden Erfindung ist es ein einfaches und universell für jede Person einsetzbares Verfahren zur Einstufung der Form eines Kopfes bereitzustellen.

**Lösung der Aufgabe**

Gelöst wird diese Aufgabe durch ein Verfahren mit den Merkmalen des Anspruchs 1. Vorteilhafte Ausführungsformen und Weiterbildungen der vorliegenden Erfindung ergeben sich aus den Unteransprüchen.

Das erfindungsgemäße Verfahren zur Einstufung der Kopfform umfassend folgende Schritte:

Zunächst wird die Länge wenigstens eines Paares Schädeldiagonalen $SD\alpha 1$ und $SD\alpha 2$ erfasst. Dann

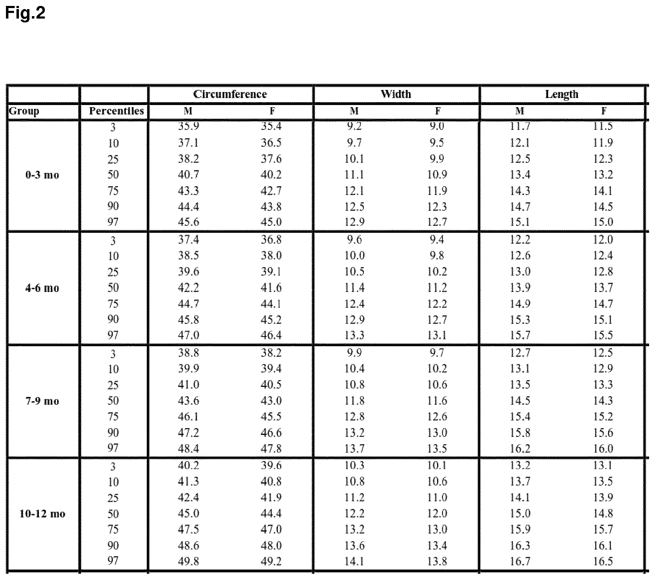

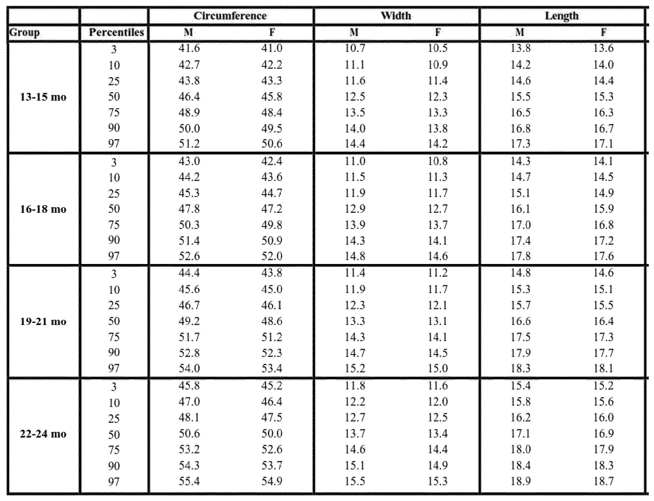

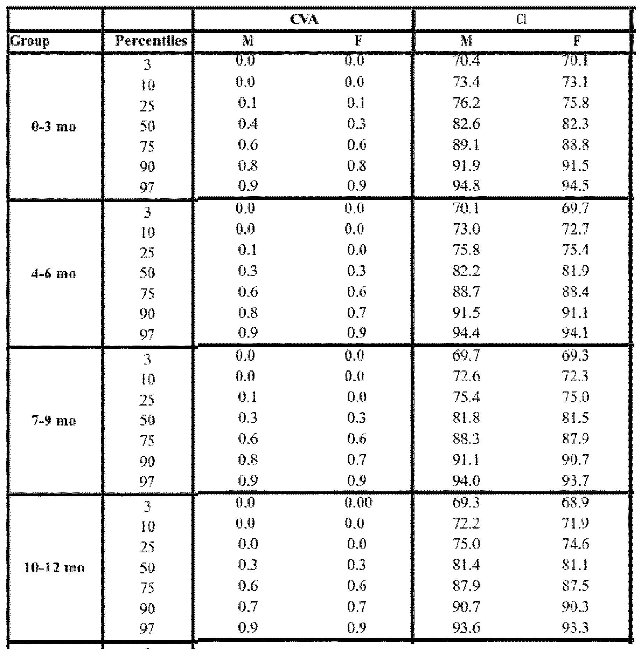

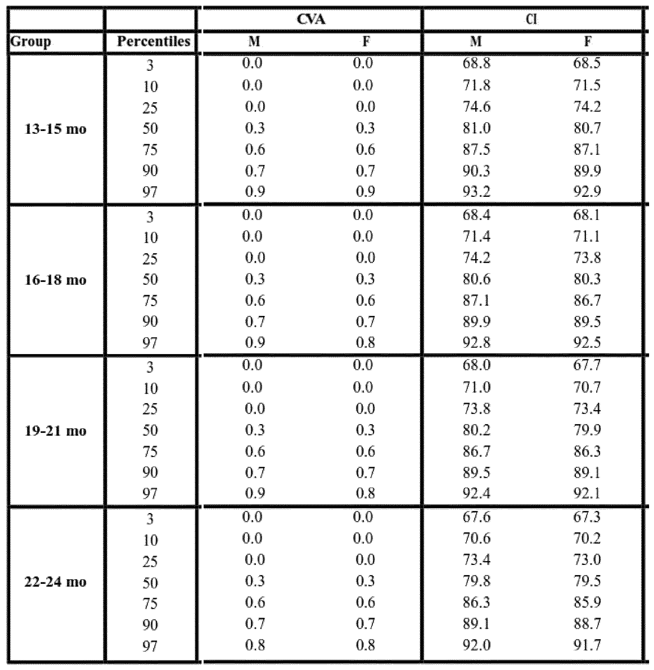

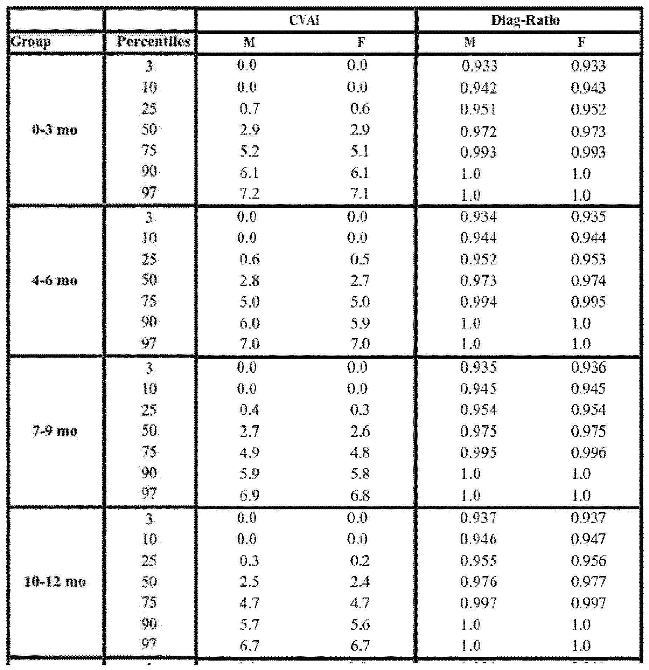

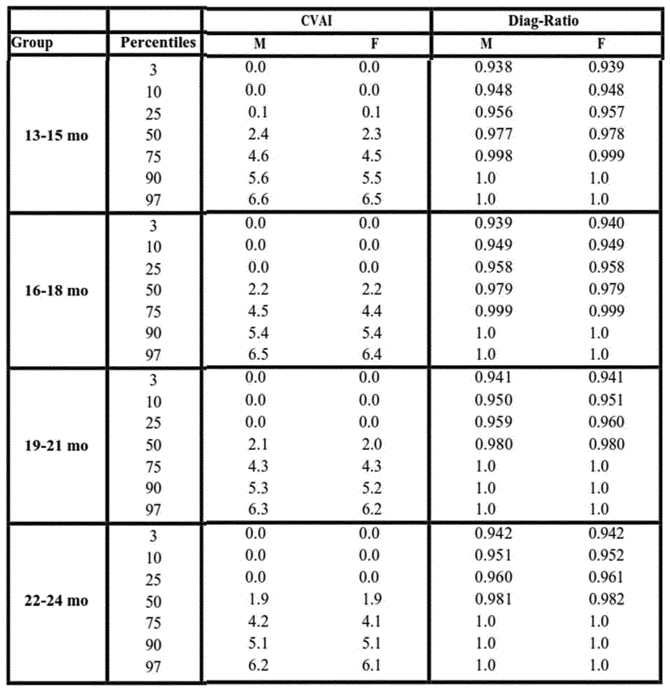

wird wenigstens einer der Kopfparameter Diag_Ratio_$\alpha$ und/oder CVA und/oder CVAI aus der Länge wenigstens eines Paares der Schädeldiagonalen SD$\alpha$1 und SD$\alpha$2 berechnet. Anschließend erfolgt ein Vergleich der Kopfparameter Diag_Ratio_$\alpha$ und/oder CVA und/oder CVAI mit den neuartigen Kopfparameternormwerten gemäß Fig.2. Dann erfolgt die Einstufung der Kopfform je nach Abweichung des/der Kopfparameter Diag_Ratio_$\alpha$ und/oder CVA und/oder CVAI von den Kopfparameternormwerten gemäß normativen Perzentilen in eine von wenigstens 3 Kategorien, wobei die Kopfform in die Kategorie "normal" eingestuft wird, wenn alle Werte der Kopfparameter zwischen der 10% und der 90% Perzentile liegen, die Kopfform in die Kategorie "moderat deformiert" eingestuft wird, wenn wenigstens ein Wert außerhalb der 10% und der 90% Perzentile liegt, aber alle Werte der Kopfparameter zwischen der 3% und der 97% Perzentile liegen oder in die Kategorie "schwer deformiert" eingestuft wird, wenn wenigstens ein Kopfparameter außerhalb der 3% und der 97% Perzentile liegt. Die Grenzwerte sind dabei vom Alter und dem Geschlecht der zu untersuchenden Person abhängig.

Hier ist ein Beispiel eine solche Einstufung:

Der Kopf eines 4 Monate alten männlichen Kleinkindes weist die Kopfparameter CVA =0,5 CVAI=3,8 und Diag_Ratio_$\alpha$=0,962 auf. Diese Werte werden nun mit den Kopfparameternormwerten für die verschiedenen Kopfparameter wie sie beispielsweise in Fig.2 angegeben werden verglichen.

Für den Kopfparameter CVA liegt der Kopfparameternormwert für die 10% Perzentile bei 0,0. Dieser Wert wird überschritten. Der Kopfparameternormwert für die 90% Perzentile liegt bei 0.8. Dieser Wert wird nicht überschritten. Der Wert für den Kopfparameter CVAI liegt also zwischen der 10% Perzentile und der 90% Perzentile.

Für den Kopfparameter CVAI liegt der Kopfparameternormwert für die 10% Perzentile bei 0,00. Dieser Wert wird nicht unterschritten. Der Kopfparameternormwert für die 90% Perzentile liegt bei 6. Dieser Wert wird nicht überschritten. Der Wert für den Kopfparameter CVAI liegt also zwischen der 10% Perzentile und der 90% Perzentile.

Für den Kopfparameter Diag_Ratio_$\alpha$ liegt der Kopfparameternormwert liegt der Kopfparameternormwert für die 10% Perzentile bei 0,944. Dieser Wert wird überchritten. Der Kopfparameternormwert für die 90% Perzentile liegt bei 1. Dieser Wert wird überschritten. Der Wert für den Kopfparameter Diag_Ratio_$\alpha$ liegt also zwischen der 10% Perzentile und der 90% Perzentile.

Die Auswertung ergibt nun, dass alle Kopfparameter der zwischen der 10% Perzentile und der 90% Perzentile liegen.

Damit erfolgt eine Einstufung des Kopfes in die Kategorie "normal".

In einer zweiten Ausführungsform des erfindungsgemäßen Verfahrens, erfolgt die Einstufung der Kopfform nur aus dem Wert des Kopfparameters Diag_Ratio_$\alpha$. Dies hat den Vorteil, dass keine Messung der absoluten Länge der Schädeldiagonalen SD$\alpha$1 und SD$\alpha$2 benötigt wird, sondern nur das Verhältnis dieser Längen zur Einstufung der Kopfform benötigt wird.

Im Folgenden wird nun eine Vorrichtung zur Durchführung des erfindungsgemäßen Verfahrens beschrieben:

Die Vorrichtung zur Durchführung des erfindungsgemäßen Verfahrens zur Erfassung der Kopfform umfasst wenigstens ein Erfassungsmittel zur Erfassung der Länge wenigstens eines Paares der Schädeldiagonalen SD$\alpha$1 und SD$\alpha$2.

Die Erfassung der Schädeldiagonalen erfolgt durch das Erfassungsmittel. Eine verbreitete Methode, um Deformationen des Schädels zu untersuchen ist die dreidimensionale Fotografie (3D-Photogrammetrie). Die Sammlung von Photoscans zahlreicher gesunder Individuen kann dabei als zuverlässiges und geeignetes Instrument für die Definition der kraniofazialen Kopfparameternormwerte dienen. Allerdings sind die finanziellen und instrumentellen Ressourcen für die Aufnahme dreidimensionaler Fotos nicht überall verfügbar. Im Gegensatz dazu sind anthropometrische Messungen, die nur zweidimensionale Änderungen in einem dreidimensionalen Körper zeigen, weit verbreitet, hoch reproduzierbar, kostengünstig und einfach zu bedienen. Vorzugsweise ist das Erfassungsmittel wie z.B. eine Kamera während der Messung in einem festen Abstand beispielsweise wenigstens 30 cm vom Kopf entfernt angeordnet. Für eine verzerrungsfreie Messung sollte das Erfassungsmittel bei der Messung der Diagonalen in einem fest vorgegebenen Winkel zum Kopf, vorzugsweise senkrecht von oben angeordnet sein.

Weiterhin umfasst die Vorrichtung ein Eingabemittel zur Eingabe der für die Einstufung der Kopfform notwendigen Personenparameter wie beispielsweise Alter und Geschlecht der zu untersuchenden Person. Bei frühgeborenen Kindern wird vorzugsweise das Alter des Kindes vom errechneten Geburtstermin verwendet. So wird bei einem 4 Monate alten Kind, das einen Monat vor dem errechneten Geburtstermin errechneten Termin geboren wurde, vorzugsweise der Kopfparameternormwert eines 3 Monate alten Kindes verwendet.

Sowohl das Erfassungsmittel als auch das Eingabemittel sind dabei so ausgebildet, dass sie die erfassten Daten des Erfassungsmittels bzw. die Eingegebenen Parameter an ein Auswertemittel übertragen können. Dies kann über eine feste Datenleitung oder Drahtlos beispielsweise über eine Funkverbindung erfolgen.

Weiterhin umfasst die Vorrichtung ein Auswertemittel zur Berechnung der Länge wenigstens eines Paares Schädeldiagonalen SD$\alpha$1 und SD$\alpha$2 und wenigs-

tens eines der Kopfparameter Diag_Ratio_$\alpha$ und/oder CVA und/oder CVAI aus den Daten des Erfassungsmittels über die Länge wenigstens eines Paares der Schädeldiagonalen SD$\alpha$1 und SD$\alpha$2, zum Vergleichen des Kopfparameters mit dem neuartigen Kopfparameternormwerten (wie beispielsweise in Abbildung Fig.2 gezeigt) sowie zur Einstufung der Kopfform je nach Abweichung von den Kopfparameternormwerten für die Kopfparameter. Die durch das Erfassungsmittel ermittelten Messwerte werden dabei durch das Auswertemittel dann mit dem alters- und geschlechtsgebundenen Kopfparameternormwerten gemäß normativer Perzentilen für die entsprechenden Parameter verglichen, um eine Einschätzung der Kopfform bezogen auf eine mögliche Schädeldeformität insbesondere von deren Schwere zu erhalten.

Diese Kopfparameternormwerte sind dabei in einer internen Datenbank des Auswertemittels hinterlegt oder kann durch einen Datenaustausch mit einer externen Datenbank beispielsweise über ein Netzwerk durch das Auswertemittel bezogen werden. Dazu weist das Auswertemittel vorzugsweise eine Schnittstelle zum elektronischen Datenaustausch (engl. electronic data interchange, EDI) auf. Als Auswertemittel dient dabei ein Gerät zur elektronischen Datenverarbeitung z.B. ein Computer oder ein Smartphone mit entsprechender Recheneinheit. Das Auswertemittel ist weiterhin so ausgebildet, dass es die Ergebnisse der Einstufung an ein Ausgabemittel übermitteln kann.

Weiterhin umfasst die Vorrichtung ein Ausgabemittel zur Ausgabe des Ergebnisses der Einstufung des Auswertemittels.

Die Ausgabe erfolgt dabei bevorzugt optisch und/oder akustisch. Als Ausgabemittel können dabei ein Lautsprecher und/oder ein Bildschirm dienen.

Hierbei ist das Auswertemittel so ausgebildet, dass die Einstufung der Kopfform je nach Abweichung von den Normwerten für die Kopfparameter in eine von wenigstens 3 Kategorien erfolgen kann. Eine feinere Unterteilung ist möglich.

Ergänzend dazu weist die Vorrichtung noch Schnittstellen zum Datenaustausch zwischen dem Erfassungsmittel, dem Auswertemittel und dem Ausgabemittel auf. Dieser kann über Datenleitungen oder drahtlos (z.B. über Funk) erfolgen.

In einer alternativen Ausführungsform umfasst die Vorrichtung weiterhin noch einen Kalibrierkörper.

Dieser Kalibrierkörper ist dabei so ausgebildet, dass er eine feste räumliche Ausdehnung und Form besitzt, welche vor der Bestimmung der Kopfform vermessen werden kann. Der Kalibrierkörper ist weiterhin so ausgebildet, dass er bei der Erfassung der Kopfform mit dem Erfassungsmittel ebenfalls mit erfasst werden kann. Über den Kalibrierkörper kann elektronisch der Winkel und die Entfernung zum fotografierten und zu vermessenden Kopf festgelegt werden.

Das Arbeitsverfahren dieser Vorrichtung besteht vorzugsweise aus den folgenden Schritten

I. Erfassung und Messung der Länge wenigstens eines ersten Paares Schädeldiagonalen SD$\alpha$1 und SD$\alpha$2 mit dem Erfassungsmittel

II. Eingabe der für die Einstufung der Kopfform notwendigen Parameter der zu untersuchenden Person

III. Berechnung wenigstens eines Kopfparameters Diag_Ratio_$\alpha$, CVA und/oder CVAI aus den Daten des Erfassungsmittels über die Länge wenigstens eines ersten Paares Schädeldiagonalen SD$\alpha$1 und SD$\alpha$2

IV. Vergleich der erhobenen Messwerte für die Kopfparameter mit Kopfparameternormwerten Fig.2 mit dem Auswertemittel

V. Einstufung der Kopfform je nach Abweichung von den Kopfparameternormwerte für die Kopfparameter mit der Auswerteeinheit und Übermittlung der Ergebnisse der Einstufung an ein Ausgabemittel

VI. Ausgabe des Ergebnisses der Einstufung mit einem Ausgabemittel

In einem alternativen Arbeitsverfahren erfolgt vor dem schritt I in einem zusätzlichen Schritt Ia der Vergleich der Länge wenigstens eines ersten Paares Schädeldiagonalen SD$\alpha$1 und SD$\alpha$2 mit einer bekannten räumlichen Ausdehnung eines Kalibrierkörpers.

Wenn dabei beispielsweise ein runder Kalibrierkörper einen bekannten Durchmesser (z.B. 25 mm) hat, reicht es für die Schädelmessung, das Verhältnis der Diagonalenlänge des Schädels zur Diagonalenlänge des Kalibrierkörpers zu vergleichen.

Bei einem Durchmesser von 25 mm und einem gemessenen Verhältnis 1:4 beträgt die Diagonalenlänge 10,0 cm

Weiterhin können bei einer bekannten Form des Kalibrierkörpers (z.B. rund) auch Verzerrungen bei der Messung z.B. dreidimensionaler Fotografie ermittelt werden. Das ermöglicht es den Winkel und die Entfernung beim der Erfassung (z.B. der Anfertigen von Fotografien) zu standardisieren wird und Messungen von Absolutwerten einfach und zuverlässig vorzunehmen.

Normative Daten der Kopfformparameter für das erste Lebensjahr sind bekannt und werden weltweit von pädiatrischen und kraniofazialen Universitätszentren eingesetzt. Für das zweite Lebensjahr wurden zusätzliche Perzentile durch neuere Messreihen generiert, da die Therapien zu Korrektur der Kopfform in der Regel die ersten 12 Monate übersteigen. Die Werte hierzu sind in der Tabelle in der Abbildung Fig.2 angegeben. Diese Perzentile spiegeln Veränderungen der Schädelgewölbeform während der ersten zwei Lebensjahre wieder und ermöglichen eine genaue alters- und geschlechtsbezogene Einstufung der kraniofazialen Deformität vor, während und nach jeder physiotherapeutischen Intervention, Helmtherapie oder Nichttherapie.

Die Verwendung von normativen Perzentilen, erlauben es sowohl Kopfdeformitäten, die sich aus externen Positionskräften ergeben, als auch diejenigen, die aus Craniosynostosen resultieren, auf einfache und standardisierte Weise zu überwachen. Die Verwendung von normativen Perzentilen kann dazu beitragen, objektive Therapieentscheidungen zu treffen, den Verlauf der Kopfform zu verfolgen und die Wirksamkeit jeglicher Art von Intervention zu beurteilen. Darüber hinaus sind die Perzentilkurven für die Eltern des Patienten sehr erläuternd, wenn sie eine informierte Zustimmung zur Behandlung erhalten, eine Reaktion auf eine Therapie zeigen und das endgültige Ergebnis zeigen. Darüber hinaus ermöglicht eine nach diesem Ansatz arbeitende Vorrichtung den Vergleich von behandelten und unbehandelten Säuglingen und die Bewertung der Schädeldeformität während künftiger Studien.

Nach einem weiteren Aspekt betrifft die Erfindung ferner ein Computerprogramm zur Messung und Einstufung von Schädeldeformitäten mit der erfindungsgemäßen Vorrichtung. Das Computerprogramm ist insbesondere in einem Auswertemittel implementierbar und dient der rechnerischen bzw. computergestützten Analyse der mit dem Erfassungsmittel aufgenommenen Bilder.

**Abbildungslegende**

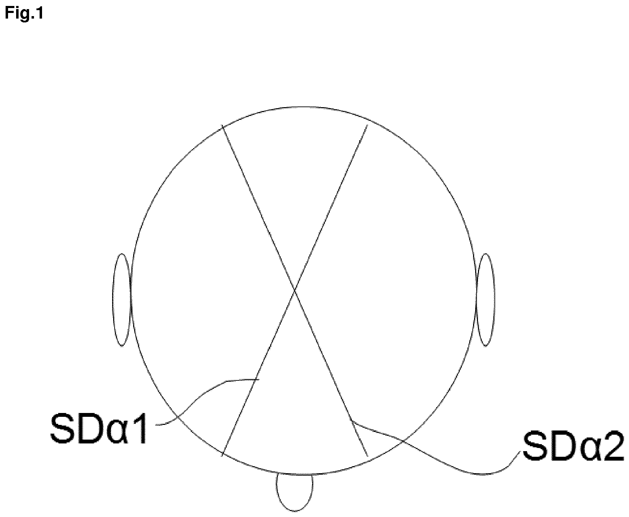

Fig. 1 Schematische Darstellung eines zu untersuchenden Kopfes

Fig.2 Normwerte für die Perzentile der Kopfgrößenparameter bei Kindern während der ersten 2 Jahre des Lebens, welche an 748 gesunden kaukasischstämmige Kindern gemessen wurde. Die Längenangaben sind hier in cm angegeben.

M steht für die gruppe männliche Kinder. F steht für die Gruppe weibliche Kinder. In der Spalte Group sind die einzelnen Altersgruppen von Kindern.

**Patentansprüche**

1. Verfahren zur Einstufung der Kopfform umfassend folgende Schritte:

I. Erfassung der Länge wenigstens eines Paares der Schädeldiagonalen $SD\alpha1$ und $SD\alpha2$

II. Berechnung der Kopfparameter $Diag\_Ratio\_\alpha$ und/oder CVA und/oder CVAI aus der Länge wenigstens eines Paares der Schädeldiagonalen $SD\alpha1$ und $SD\alpha2$

III. Vergleich der Kopfparameter $Diag\_Ratio\_\alpha$ und/oder CVA und/oder CVAI mit Kopfparameternormwerten

IV. Einstufung der Kopfform je nach Abweichung des/der Kopfparameter $Diag\_Ratio\_\alpha$ und/oder CVA und/oder CVAI von Kopfparameternormwerten gemäß normativen Perzentilen in eine von wenigstens 3 Kategorien, wobei die Kopfform in die Kategorie "normal" eingestuft wird, wenn alle Werte der Kopfparameter zwischen der 10% und der 90% Perzentile liegen, die Kopfform in die Kategorie "moderat deformiert" eingestuft wird, wenn wenigstens ein Wert außerhalb der 10% und der 90% Perzentile liegt, aber alle Werte der Kopfparameter zwischen der 3% und der 97% Perzentile liegen oder in die Kategorie "schwer deformiert" eingestuft wird, wenn wenigstens ein Kopfparameter außerhalb der 3% und der 97% Perzentile liegt.

2. Verfahren zur Einstufung der Kopfform gemäß Anspruch1, **dadurch gekennzeichnet, dass** die Einstufung der Kopfform nur aus dem Wert des Kopfparameters $Diag\_Ratio\_\alpha$ erfolgt.

3. Vorrichtung zur Einstufung der Kopfform mit einem Verfahren gemäß einem der vorigen Ansprüche umfassend

• wenigstens ein Erfassungsmittel zur Erfassung der Länge wenigstens eines Paares der Schädeldiagonalen $SD\alpha1$ und $SD\alpha2$
• wenigstens ein Eingabemittel zur Eingabe der für die Einstufung der Kopfform notwendigen Personenparameter der zu untersuchenden Person
• wenigstens ein Auswertemittel zur Berechnung wenigstens eines der Kopfparameter $Diag\_Ratio\_\alpha$ und/oder CVA und/oder CVAI aus der Länge wenigstens eines Paares der Schädeldiagonalen $SD\alpha1$ und $SD\alpha2$, zum Vergleichen wenigstens eines Kopfparameters mit Kopfparameternormwerten gemäß normativen Perzentilen, zur Einstufung der Kopfform sowie zur Weiterleitung des Ergebnisses der Einstufung an ein Ausgabemittel, wobei die Kopfparameternormwerte dabei in einer internen Datenbank des Auswertemittels hinterlegt durch einen Datenaustausch des Auswertemittels mit einer externen Datenbank beziehbar sind
• wenigstens ein Ausgabemittel zur Anzeige des Ergebnisses der Einstufung des Auswertmittels **dadurch gekennzeichnet, dass** das Auswertemittel so ausgebildet ist, dass die Einstufung der Kopfform je nach Abweichung von den Kopfparameternormwerten gemäß normativen Perzentilen in eine von wenigstens 3 Kategorien erfolgen kann, wobei die Kopfform in die Kategorie "normal" eingestuft wird, wenn alle Werte der Kopfparameter zwischen der 10% und der 90% Perzentile liegen, die Kopfform in die Kategorie "moderat deformiert" eingestuft wird, wenn wenigstens ein Wert außerhalb der 10% und der 90% Perzentile liegt, alle Werte der Kopfparameter zwischen der 3% und der 97% Perzentile liegen oder in die Kategorie "schwer deformiert" eingestuft wird, wenn wenigstens ein Kopfparameter außerhalb der 3% und der 97% Perzentile liegt.

4. Vorrichtung zur Einstufung der Kopfform gemäß Anspruch 3 **dadurch gekennzeichnet, dass** diese weiterhin noch einen Kalibrierkörper umfasst, welche eine feste räumliche Ausdehnung und Form besitzt, welche vor der Bestimmung der Kopfform vermessen werden kann und welcher weiterhin so ausgebildet ist, dass er bei der Erfassung der Kopfform mit dem Erfassungsmittel ebenfalls mit erfasst werden kann.

5. Verfahren zum Betrieb einer Vorrichtung zur Einstufung der Kopfform gemäß Anspruch 3 oder 4 für ein Verfahren zur Einstufung der Kopfform gemäß Anspruch 1 oder 2 umfassend folgende Schritte:

I. Erfassung wenigstens eines Paares Schädeldiagonalen SD$\alpha$1 und SD$\alpha$2 mit dem Erfassungsmittel

II. Eingabe der für die Einstufung der Kopfform notwendigen Parameter der zu untersuchenden Person

III. Berechnung der Länge wenigstens eines Paares Schädeldiagonalen SD$\alpha$1 und SD$\alpha$2 und wenigstens eines Kopfparameters Diag_Ratio_$\alpha$ und/oder, CVA und/oder CVAI aus den Daten des Erfassungsmittels über die Länge wenigstens eines Paares der Schädeldiagonalen SD$\alpha$1 und SD$\alpha$2

IV. Vergleich der erhobenen Messwerte für die Kopfparameter mit den Kopfparameternormwerten gemäß normativen Perzentilen mit dem Auswertemittel

V. Einstufung der Kopfform je nach Abweichung von Kopfparameternormwerten mit dem Auswertemittel und Übertragung der Daten der Einstufung an ein Ausgabemittel

VI. Ausgabe des Ergebnisses der Einstufung mit einem Ausgabemittel

6. Verfahren gemäß Anspruch 5 **dadurch gekennzeichnet, dass** einem zusätzlichen Schritt Ia umfasst, wobei die Länge wenigstens eines ersten Paares Schädeldiagonalen SD$\alpha$1 und SD$\alpha$2 mit einer bekannten räumlichen Ausdehnung eines Kalibrierkörpers verglichen wird.

7. Computerprogramm zur Durchführung eines Verfahrens gemäß Anspruch 1 oder 2 mit einer Vorrichtung gemäß Anspruch 3 oder 4.

**Fig.1**

SDα1

SDα2

**Fig.2**

| Group | Percentiles | Circumference | | Width | | Length | |
|---|---|---|---|---|---|---|---|
| | | M | F | M | F | M | F |
| 0-3 mo | 3 | 35.9 | 35.4 | 9.2 | 9.0 | 11.7 | 11.5 |
| | 10 | 37.1 | 36.5 | 9.7 | 9.5 | 12.1 | 11.9 |
| | 25 | 38.2 | 37.6 | 10.1 | 9.9 | 12.5 | 12.3 |
| | 50 | 40.7 | 40.2 | 11.1 | 10.9 | 13.4 | 13.2 |
| | 75 | 43.3 | 42.7 | 12.1 | 11.9 | 14.3 | 14.1 |
| | 90 | 44.4 | 43.8 | 12.5 | 12.3 | 14.7 | 14.5 |
| | 97 | 45.6 | 45.0 | 12.9 | 12.7 | 15.1 | 15.0 |
| 4-6 mo | 3 | 37.4 | 36.8 | 9.6 | 9.4 | 12.2 | 12.0 |
| | 10 | 38.5 | 38.0 | 10.0 | 9.8 | 12.6 | 12.4 |
| | 25 | 39.6 | 39.1 | 10.5 | 10.2 | 13.0 | 12.8 |
| | 50 | 42.2 | 41.6 | 11.4 | 11.2 | 13.9 | 13.7 |
| | 75 | 44.7 | 44.1 | 12.4 | 12.2 | 14.9 | 14.7 |
| | 90 | 45.8 | 45.2 | 12.9 | 12.7 | 15.3 | 15.1 |
| | 97 | 47.0 | 46.4 | 13.3 | 13.1 | 15.7 | 15.5 |
| 7-9 mo | 3 | 38.8 | 38.2 | 9.9 | 9.7 | 12.7 | 12.5 |
| | 10 | 39.9 | 39.4 | 10.4 | 10.2 | 13.1 | 12.9 |
| | 25 | 41.0 | 40.5 | 10.8 | 10.6 | 13.5 | 13.3 |
| | 50 | 43.6 | 43.0 | 11.8 | 11.6 | 14.5 | 14.3 |
| | 75 | 46.1 | 45.5 | 12.8 | 12.6 | 15.4 | 15.2 |
| | 90 | 47.2 | 46.6 | 13.2 | 13.0 | 15.8 | 15.6 |
| | 97 | 48.4 | 47.8 | 13.7 | 13.5 | 16.2 | 16.0 |
| 10-12 mo | 3 | 40.2 | 39.6 | 10.3 | 10.1 | 13.2 | 13.1 |
| | 10 | 41.3 | 40.8 | 10.8 | 10.6 | 13.7 | 13.5 |
| | 25 | 42.4 | 41.9 | 11.2 | 11.0 | 14.1 | 13.9 |
| | 50 | 45.0 | 44.4 | 12.2 | 12.0 | 15.0 | 14.8 |
| | 75 | 47.5 | 47.0 | 13.2 | 13.0 | 15.9 | 15.7 |
| | 90 | 48.6 | 48.0 | 13.6 | 13.4 | 16.3 | 16.1 |
| | 97 | 49.8 | 49.2 | 14.1 | 13.8 | 16.7 | 16.5 |

| Group | Percentiles | Circumference | | Width | | Length | |
|---|---|---|---|---|---|---|---|
| | | M | F | M | F | M | F |
| **13-15 mo** | 3 | 41.6 | 41.0 | 10.7 | 10.5 | 13.8 | 13.6 |
| | 10 | 42.7 | 42.2 | 11.1 | 10.9 | 14.2 | 14.0 |
| | 25 | 43.8 | 43.3 | 11.6 | 11.4 | 14.6 | 14.4 |
| | 50 | 46.4 | 45.8 | 12.5 | 12.3 | 15.5 | 15.3 |
| | 75 | 48.9 | 48.4 | 13.5 | 13.3 | 16.5 | 16.3 |
| | 90 | 50.0 | 49.5 | 14.0 | 13.8 | 16.8 | 16.7 |
| | 97 | 51.2 | 50.6 | 14.4 | 14.2 | 17.3 | 17.1 |
| **16-18 mo** | 3 | 43.0 | 42.4 | 11.0 | 10.8 | 14.3 | 14.1 |
| | 10 | 44.2 | 43.6 | 11.5 | 11.3 | 14.7 | 14.5 |
| | 25 | 45.3 | 44.7 | 11.9 | 11.7 | 15.1 | 14.9 |
| | 50 | 47.8 | 47.2 | 12.9 | 12.7 | 16.1 | 15.9 |
| | 75 | 50.3 | 49.8 | 13.9 | 13.7 | 17.0 | 16.8 |
| | 90 | 51.4 | 50.9 | 14.3 | 14.1 | 17.4 | 17.2 |
| | 97 | 52.6 | 52.0 | 14.8 | 14.6 | 17.8 | 17.6 |
| **19-21 mo** | 3 | 44.4 | 43.8 | 11.4 | 11.2 | 14.8 | 14.6 |
| | 10 | 45.6 | 45.0 | 11.9 | 11.7 | 15.3 | 15.1 |
| | 25 | 46.7 | 46.1 | 12.3 | 12.1 | 15.7 | 15.5 |
| | 50 | 49.2 | 48.6 | 13.3 | 13.1 | 16.6 | 16.4 |
| | 75 | 51.7 | 51.2 | 14.3 | 14.1 | 17.5 | 17.3 |
| | 90 | 52.8 | 52.3 | 14.7 | 14.5 | 17.9 | 17.7 |
| | 97 | 54.0 | 53.4 | 15.2 | 15.0 | 18.3 | 18.1 |
| **22-24 mo** | 3 | 45.8 | 45.2 | 11.8 | 11.6 | 15.4 | 15.2 |
| | 10 | 47.0 | 46.4 | 12.2 | 12.0 | 15.8 | 15.6 |
| | 25 | 48.1 | 47.5 | 12.7 | 12.5 | 16.2 | 16.0 |
| | 50 | 50.6 | 50.0 | 13.7 | 13.4 | 17.1 | 16.9 |
| | 75 | 53.2 | 52.6 | 14.6 | 14.4 | 18.0 | 17.9 |
| | 90 | 54.3 | 53.7 | 15.1 | 14.9 | 18.4 | 18.3 |
| | 97 | 55.4 | 54.9 | 15.5 | 15.3 | 18.9 | 18.7 |

| Group | Percentiles | CVA | | CI | |
|---|---|---|---|---|---|
| | | M | F | M | F |
| 0-3 mo | 3 | 0.0 | 0.0 | 70.4 | 70.1 |
| | 10 | 0.0 | 0.0 | 73.4 | 73.1 |
| | 25 | 0.1 | 0.1 | 76.2 | 75.8 |
| | 50 | 0.4 | 0.3 | 82.6 | 82.3 |
| | 75 | 0.6 | 0.6 | 89.1 | 88.8 |
| | 90 | 0.8 | 0.8 | 91.9 | 91.5 |
| | 97 | 0.9 | 0.9 | 94.8 | 94.5 |
| 4-6 mo | 3 | 0.0 | 0.0 | 70.1 | 69.7 |
| | 10 | 0.0 | 0.0 | 73.0 | 72.7 |
| | 25 | 0.1 | 0.0 | 75.8 | 75.4 |
| | 50 | 0.3 | 0.3 | 82.2 | 81.9 |
| | 75 | 0.6 | 0.6 | 88.7 | 88.4 |
| | 90 | 0.8 | 0.7 | 91.5 | 91.1 |
| | 97 | 0.9 | 0.9 | 94.4 | 94.1 |
| 7-9 mo | 3 | 0.0 | 0.0 | 69.7 | 69.3 |
| | 10 | 0.0 | 0.0 | 72.6 | 72.3 |
| | 25 | 0.1 | 0.0 | 75.4 | 75.0 |
| | 50 | 0.3 | 0.3 | 81.8 | 81.5 |
| | 75 | 0.6 | 0.6 | 88.3 | 87.9 |
| | 90 | 0.8 | 0.7 | 91.1 | 90.7 |
| | 97 | 0.9 | 0.9 | 94.0 | 93.7 |
| 10-12 mo | 3 | 0.0 | 0.00 | 69.3 | 68.9 |
| | 10 | 0.0 | 0.0 | 72.2 | 71.9 |
| | 25 | 0.0 | 0.0 | 75.0 | 74.6 |
| | 50 | 0.3 | 0.3 | 81.4 | 81.1 |
| | 75 | 0.6 | 0.6 | 87.9 | 87.5 |
| | 90 | 0.7 | 0.7 | 90.7 | 90.3 |
| | 97 | 0.9 | 0.9 | 93.6 | 93.3 |

|  |  | CVA | | CI | |
|---|---|---|---|---|---|
| Group | Percentiles | M | F | M | F |
| 13-15 mo | 3 | 0.0 | 0.0 | 68.8 | 68.5 |
|  | 10 | 0.0 | 0.0 | 71.8 | 71.5 |
|  | 25 | 0.0 | 0.0 | 74.6 | 74.2 |
|  | 50 | 0.3 | 0.3 | 81.0 | 80.7 |
|  | 75 | 0.6 | 0.6 | 87.5 | 87.1 |
|  | 90 | 0.7 | 0.7 | 90.3 | 89.9 |
|  | 97 | 0.9 | 0.9 | 93.2 | 92.9 |
| 16-18 mo | 3 | 0.0 | 0.0 | 68.4 | 68.1 |
|  | 10 | 0.0 | 0.0 | 71.4 | 71.1 |
|  | 25 | 0.0 | 0.0 | 74.2 | 73.8 |
|  | 50 | 0.3 | 0.3 | 80.6 | 80.3 |
|  | 75 | 0.6 | 0.6 | 87.1 | 86.7 |
|  | 90 | 0.7 | 0.7 | 89.9 | 89.5 |
|  | 97 | 0.9 | 0.8 | 92.8 | 92.5 |
| 19-21 mo | 3 | 0.0 | 0.0 | 68.0 | 67.7 |
|  | 10 | 0.0 | 0.0 | 71.0 | 70.7 |
|  | 25 | 0.0 | 0.0 | 73.8 | 73.4 |
|  | 50 | 0.3 | 0.3 | 80.2 | 79.9 |
|  | 75 | 0.6 | 0.6 | 86.7 | 86.3 |
|  | 90 | 0.7 | 0.7 | 89.5 | 89.1 |
|  | 97 | 0.9 | 0.8 | 92.4 | 92.1 |
| 22-24 mo | 3 | 0.0 | 0.0 | 67.6 | 67.3 |
|  | 10 | 0.0 | 0.0 | 70.6 | 70.2 |
|  | 25 | 0.0 | 0.0 | 73.4 | 73.0 |
|  | 50 | 0.3 | 0.3 | 79.8 | 79.5 |
|  | 75 | 0.6 | 0.6 | 86.3 | 85.9 |
|  | 90 | 0.7 | 0.7 | 89.1 | 88.7 |
|  | 97 | 0.8 | 0.8 | 92.0 | 91.7 |

|  |  | CVAI | | Diag-Ratio | |
|---|---|---|---|---|---|
| Group | Percentiles | M | F | M | F |
| 0-3 mo | 3 | 0.0 | 0.0 | 0.933 | 0.933 |
|  | 10 | 0.0 | 0.0 | 0.942 | 0.943 |
|  | 25 | 0.7 | 0.6 | 0.951 | 0.952 |
|  | 50 | 2.9 | 2.9 | 0.972 | 0.973 |
|  | 75 | 5.2 | 5.1 | 0.993 | 0.993 |
|  | 90 | 6.1 | 6.1 | 1.0 | 1.0 |
|  | 97 | 7.2 | 7.1 | 1.0 | 1.0 |
| 4-6 mo | 3 | 0.0 | 0.0 | 0.934 | 0.935 |
|  | 10 | 0.0 | 0.0 | 0.944 | 0.944 |
|  | 25 | 0.6 | 0.5 | 0.952 | 0.953 |
|  | 50 | 2.8 | 2.7 | 0.973 | 0.974 |
|  | 75 | 5.0 | 5.0 | 0.994 | 0.995 |
|  | 90 | 6.0 | 5.9 | 1.0 | 1.0 |
|  | 97 | 7.0 | 7.0 | 1.0 | 1.0 |
| 7-9 mo | 3 | 0.0 | 0.0 | 0.935 | 0.936 |
|  | 10 | 0.0 | 0.0 | 0.945 | 0.945 |
|  | 25 | 0.4 | 0.3 | 0.954 | 0.954 |
|  | 50 | 2.7 | 2.6 | 0.975 | 0.975 |
|  | 75 | 4.9 | 4.8 | 0.995 | 0.996 |
|  | 90 | 5.9 | 5.8 | 1.0 | 1.0 |
|  | 97 | 6.9 | 6.8 | 1.0 | 1.0 |
| 10-12 mo | 3 | 0.0 | 0.0 | 0.937 | 0.937 |
|  | 10 | 0.0 | 0.0 | 0.946 | 0.947 |
|  | 25 | 0.3 | 0.2 | 0.955 | 0.956 |
|  | 50 | 2.5 | 2.4 | 0.976 | 0.977 |
|  | 75 | 4.7 | 4.7 | 0.997 | 0.997 |
|  | 90 | 5.7 | 5.6 | 1.0 | 1.0 |
|  | 97 | 6.7 | 6.7 | 1.0 | 1.0 |

| Group | Percentiles | CVAI | | Diag-Ratio | |
|---|---|---|---|---|---|
| | | M | F | M | F |
| 13-15 mo | 3 | 0.0 | 0.0 | 0.938 | 0.939 |
| | 10 | 0.0 | 0.0 | 0.948 | 0.948 |
| | 25 | 0.1 | 0.1 | 0.956 | 0.957 |
| | 50 | 2.4 | 2.3 | 0.977 | 0.978 |
| | 75 | 4.6 | 4.5 | 0.998 | 0.999 |
| | 90 | 5.6 | 5.5 | 1.0 | 1.0 |
| | 97 | 6.6 | 6.5 | 1.0 | 1.0 |
| 16-18 mo | 3 | 0.0 | 0.0 | 0.939 | 0.940 |
| | 10 | 0.0 | 0.0 | 0.949 | 0.949 |
| | 25 | 0.0 | 0.0 | 0.958 | 0.958 |
| | 50 | 2.2 | 2.2 | 0.979 | 0.979 |
| | 75 | 4.5 | 4.4 | 0.999 | 0.999 |
| | 90 | 5.4 | 5.4 | 1.0 | 1.0 |
| | 97 | 6.5 | 6.4 | 1.0 | 1.0 |
| 19-21 mo | 3 | 0.0 | 0.0 | 0.941 | 0.941 |
| | 10 | 0.0 | 0.0 | 0.950 | 0.951 |
| | 25 | 0.0 | 0.0 | 0.959 | 0.960 |
| | 50 | 2.1 | 2.0 | 0.980 | 0.980 |
| | 75 | 4.3 | 4.3 | 1.0 | 1.0 |
| | 90 | 5.3 | 5.2 | 1.0 | 1.0 |
| | 97 | 6.3 | 6.2 | 1.0 | 1.0 |
| 22-24 mo | 3 | 0.0 | 0.0 | 0.942 | 0.942 |
| | 10 | 0.0 | 0.0 | 0.951 | 0.952 |
| | 25 | 0.0 | 0.0 | 0.960 | 0.961 |
| | 50 | 1.9 | 1.9 | 0.981 | 0.982 |
| | 75 | 4.2 | 4.1 | 1.0 | 1.0 |
| | 90 | 5.1 | 5.1 | 1.0 | 1.0 |
| | 97 | 6.2 | 6.1 | 1.0 | 1.0 |

**Europäisches Patentamt**
**European Patent Office**
**Office européen des brevets**

# EUROPÄISCHER RECHERCHENBERICHT

Nummer der Anmeldung

EP 17 17 9899

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Dulcey Lima: "Asymmetrical Brachycephaly: A New Classification of Head Shape Deformity in Young Babies Dulcey Lima CO, OTR/L Orthomerica Products", , 30. Dezember 2010 (2010-12-30), XP055322335, Gefunden im Internet: URL:https://web.archive.org/web/2010123001 3537/http://www.oandp.org/publications/jop /2009/2009-07.pdf [gefunden am 2016-11-23] | 1-3,5,7 | INV. G16H50/20 |
| Y | * Seite 1, Zeile 39 - Seite 2, Zeile 4 * * Seite 3, Zeile 1 - Zeile 20 * ----- | 4,6 | |
| A | Anonymous: "actionorthosante.ca/english", , 24. Januar 2015 (2015-01-24), XP055322347, Gefunden im Internet: URL:https://web.archive.org/web/2015012404 0825/http://actionorthosante.ca/english/Pl agiocephaly-Severity-Scale.php [gefunden am 2016-11-23] * Seite 2 - Seite 4 * ----- | 1-7 | |
| Y | WO 2006/131922 A2 (DYNATOMICS LTD [IL]; IYUN EPHRAIM [IL]; HAZAN SHALOM [IL]) 14. Dezember 2006 (2006-12-14) * Abbildungen 16, 17 * * Seite 5, Zeile 25 - Zeile 27 * * Seite 13, Zeile 3 - Zeile 5 * ----- | 4,6 | |

**RECHERCHIERTE SACHGEBIETE (IPC)**

G16H

Der vorliegende Recherchenbericht wurde für alle Patentansprüche erstellt

| Recherchenort | Abschlußdatum der Recherche | Prüfer |
|---|---|---|
| München | 8. Januar 2018 | Kutzarova, Iskrena |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 17 17 9899

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-01-2018

| Im Recherchenbericht angeführtes Patentdokument | Datum der Veröffentlichung | Mitglied(er) der Patentfamilie | Datum der Veröffentlichung |
|---|---|---|---|
| WO 2006131922 A2 | 14-12-2006 | KEINE | |

Für nähere Einzelheiten zu diesem Anhang : siehe Amtsblatt des Europäischen Patentamts, Nr.12/82